# EUROPEAN PATENT SPECIFICATION

(11) **EP 0 649 634 B1**
(45) Date of publication and mention of the grant of the patent: **02.09.1998**
(21) Application number: 94307812.1
(22) Date of filing: 25.10.1994
(51) Int. Cl.: A61B 17/34

(54) **Trocar with a retractable cannula**
Trokar mit zurückziehbarer Kanüle
Trocart à cannule rétractable

(30) Priority: 26.10.1993 US 143208
(43) Date of publication of application: 26.04.1995
(73) Proprietor: ETHICON ENDO-SURGERY, Cincinnati, Ohio 45242 (US)
(72) Inventor: Young, Joseph, Loveland, Ohio 45140 (US); Schwemberger, Richard F., Cincinnati, Ohio 45257 (US); Warman, Thomas E., Cincinnati, Ohio 45140 (US)
(74) Representative: Fisher, Adrian John

(56) References cited:
- US-A- 5 236 424

## Description

### Field of the Invention

Generally this invention relates to trocars. More specifically, this invention relates to trocars which have cannulas wherein one portion of the cannula is capable of moving, and thereby being retracted with respect to another portion of the cannula.

### Background of the Invention

Trocars are instruments which are insertable into the abdomen of a patient. In their most basic sense, trocars contain a obturator, which is a rigid shaft generally containing a puncturing mechanism able to pierce tissue. This obturator is placed within a hollow cannula, which consists of a lumen which is disposed about the obturator shaft during insertion into tissue, Thereafter, the obturator may be removed and the hollow interior section of the cannula be exposed. Thereafter surgical instruments can be placed within the cannula so that endoscopic or laparoscopic surgery can take place.

In the use of such trocars, it is realized that it may be desirable to allow for adjustment of the cannula within the abdominal wall. That is, abdominal walls vary in thickness from patient to patient, and indeed even in the same patient. Moreover, even with a constant thickness of a patient's abdominal wall, it may be desirable to adjust the relative position of the cannula within the abdominal wall, so that the distal end of the cannula lies a pre-determined distance from the end of the abdominal wall. Devices have been developed such as that disclosed by US-A-5236424 in which a catheter comprises a retractable cannula. The retractable cannula allows simple adjustment of the position of the distal end within the body of a patient. However no such device has been developed to assist the positioning of a trocar within the body of the patient. For instance, if one desires to operate in or near the mesentery, just below the abdominal wall, it would be favourable for the cannula distal end to be disposed only to the extent that it is placed at the abdominal or just even with the abdominal wall.

### Summary of the Invention

Accordingly, it is an object of the invention to create a trocar with a cannula wherein the cannula can be made to assume a varying length. This is possible in a trocar comprising a cannula for use with an obturator which contains a distal portion and a proximal portion extending therefrom. The distal portion is retractable with respect to the proximal portion. The trocar further comprises an obturator insertable into said cannula, the obturator comprising a mechanism for retracting said distal portion with respect to said proximal portion. Generally, the distal portion retracts around the outside of the proximal portion. There may be disposed sealing means between the distal and proximal portions, to allow for sealing of the wall of the proximal portion with respect to the puncture, to enhance insufflation. There is further disclosed a mechanism which causes the distal portion to lock with respect to the proximal portion to maintain the cannula in a predetermined position.

The obturator contains a latching mechanism which mates with the distal portion of the cannula. When the obturator is retracted, this latching mechanism may be actuated by an actuation mechanism contained in the handle of the obturator. The latching mechanism thereafter engages the distal portion so that the obturator itself pulls the distal portion to retract with respect to the proximal portion. When the cannula is placed at its desired length, the latching mechanism is released and the obturator is able to be retracted from the trocar cannula. Thereafter, surgical instructions can be used within the cannula set to the desired varying length. Of course, this length can be changed from patient to patient, or indeed even within the same patient by use of the actuation and latching mechanisms described above.

### Description of the Drawings

This invention will be better understood in connection with the following drawings wherein:
Figure 1 is a perspective view of the present invention showing an extending obturator and cannula;
Figure 2 is a side elevation view of the cannula handle and the proximal portion of the cannula;
Figure 3 is a cross sectional view of the cannula taken across lines 3-3 of Figure 2;
Figure 4 is a partial cross-sectional view taken across lines B-B of Figure 2;
Figure 5 is an enlarged view of the mating mechanism contained on the distal end of the proximal portion of the cannula;
Figure 6 is a side view of the distal portion of the cannula;
Figure 7 is a cross-sectional view of the distal portion shown in Figure 6;
Figure 8 is a side view of the obturator handle containing the latching means described above;
Figure 9 is a view of an obturator containing a latching means exposed in order to latch with the trocar cannula; and
Figure 10 is a partial cross-section of the view of Figure 9.

### Description of the Invention

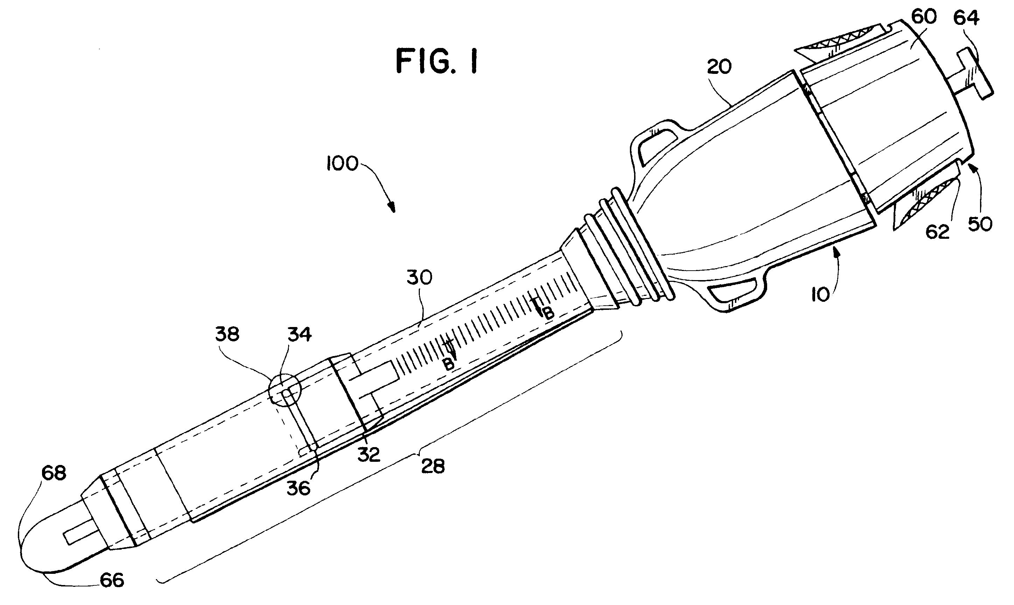

As seen in Figures 1 through 10, there is contained a trocar assembly 100 which consists of a cannula handle assembly 10 and obturator handle assembly 50. The cannula handle assembly 10 can be seen generally in Figure 1. Cannula handle assembly 10 includes a cannula handle 20, which has a hollow interior 22 for insertion of an obturator. The cannula handle assembly 10 further includes a distal end 24 which has a gripping device 26 for gripping the trocar cannula. The trocar cannula 28 consists of a proximal portion 30 projecting from the cannula handle 20 and a distal portion 32 projecting from the proximal portion 30. The relationship of the distal portion 32 and the proximal portion 30 will be better described herein.

As can be seen in Figure 1, there is contained an O-ring 34 disposed in a groove notch 36 contained at the distal end 38 of the proximal portion 30. This O-ring 34 allows for sealing between the inner diameter of the distal portion 32 and the outer diameter of the proximal portion 30.

As further seen in Figure 1, there is a obturator handle assembly 50 comprising handle 60 which contains an attachment means 62 which releasably attaches the obturator handle 60 to the cannula handle 20. There is further disclosed an actuation button 64 which will be better described below. Extending from the obturator handle 60 there is contained an obturator 66. In this instance, the obturator 66 has a blunt end 68 projecting from the distal portion 32 of the trocar cannula 28. However, it is appreciated that it is also possible to provide a sharpened tip, which is quite common for surgical trocars, without departing from the scope of this invention.

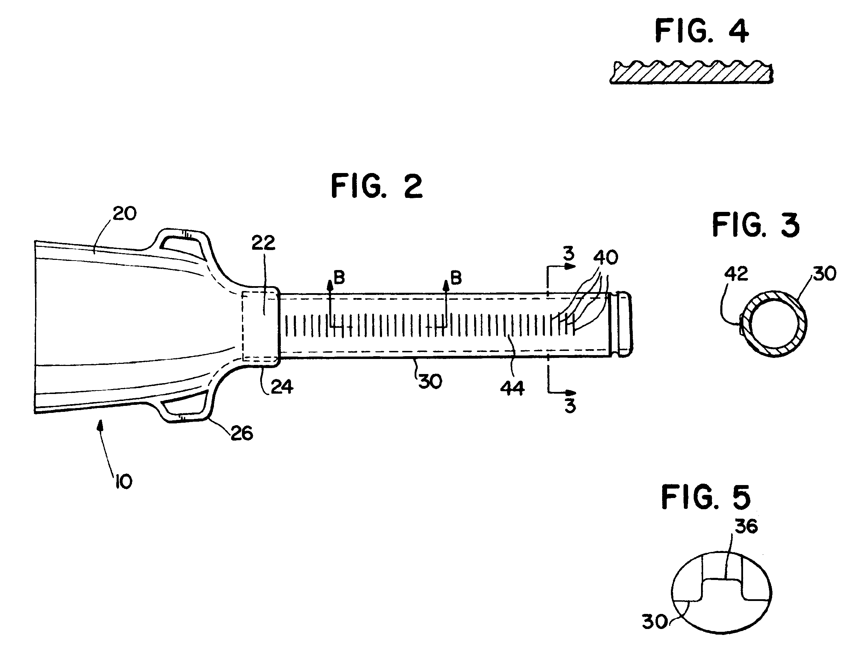

As seen in Figures 2, 3, 4, and 5, there is contained the cannula handle 20 with the proximal portion 30 extending therefrom. The proximal portion 30 is generally an extruded tube which contains a series of markings 40 which will help the user determine the varying length of the cannula 28 after it has been actuated as described below. A cross-sectional view (Figure 3) of the proximal portion 30 of the cannula 28 shows that it has a cylindrical interior and a generally cylindrical exterior. There is a flattened portion 42 contained on the proximal portion 30 which mates with the distal portion 32 of the cannula 28. This allows the distal portion 32 to ride along the keyway created by this flattened section 42, and ultimately lock thereon. As can be seen from the cross-sectional view of the exterior of the proximal portion 30 of trocar cannula 28, there are contained a series of ridges 44 corresponding to the marked lengths 40 of the trocar cannula 28. These ridges enable the distal portion 32 to lock against the proximal portion 30.

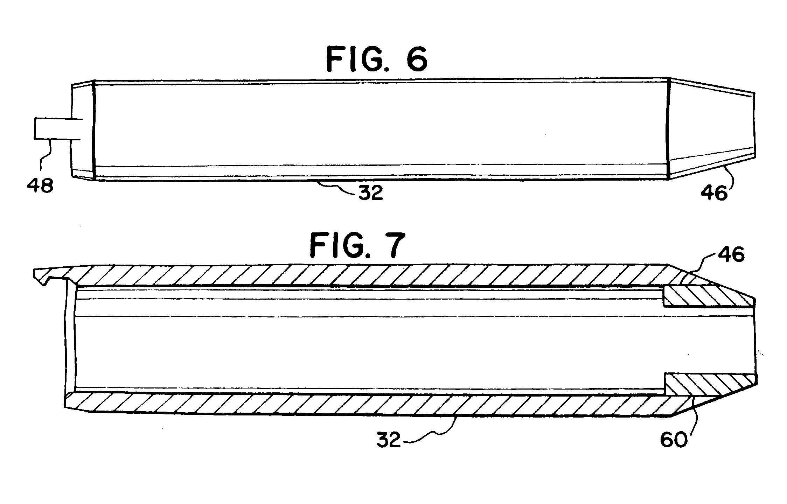

The distal portion 32 is better described in Figures 6 and 7. It consists of a generally cylindrical sleeve which contains a beveled end 46 toward its distal side. This beveled end 46 allows easier insertion within the abdominal wall. At the proximal end of distal portion 32, there is contained a ridged notch 48 which serves as a latching mechanism for this device. This ridged notch 48 can also be seen in Figure 1. The ridged notch 48 is capable of mating with the ridges 44 contained on the exterior wall of the proximal portion 30 as described above. Thus, after the trocar has been moved to a given length, this ridged notch 48 locks against the ridges 40 contained on the proximal portion 30 so that the portions 30, 32 remains in place with respect to one another.

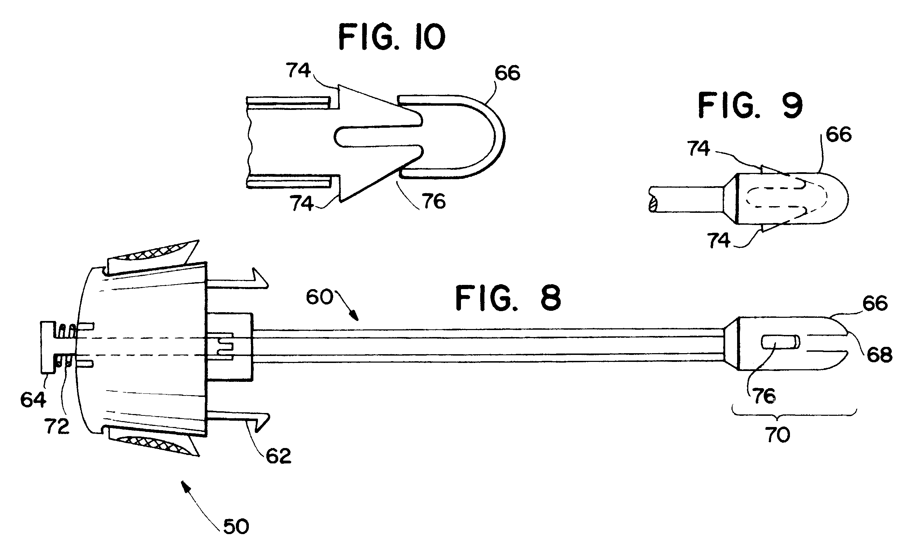

In Figures 8, 9, and 10 provide views of the obturator 50 described herein. Projected from the obturator handle 60 is the actuation or latching means 62 which latches against the cannula handle 20, as well as the obturator 66 which is inserted into the cannula handle 20 and through the cannula 28. Also contained on the obturator handle is a actuation or latch button 64 which serves as an actuating mechanism for actuating the latching device 70 contained at the distal end of the obturator 66. Optionally, there can be contained a spring 72 which biases the latch button 64 in its non-actuated position. Of course, it will be appreciated that the optional nature of the spring naturally makes such mechanism merely desirable, and not necessary to accomplish the basic functions of this invention.

On the blunt distal end 68 of the obturator 66 there is contained a blunt end 68. This blunt end is hollow and contains therein the latch mechanism 70 which will be capable of adjusting the variable length of the trocar cannula 28. This latch mechanism consist of a biased pair of teeth 74 which are exposed through window shaped openings contained in the distal end 68 of the obturator 66. This latching mechanism 70 is actuated by the latch button 64 contained at the proximal end of the obturator handle 60. It will be appreciated that when the latching button 64 is pushed distally, that is, against the obturator handle 60, the distal end latching mechanism 70 is caused to move within the hollow distal end 68 of the obturator 66. This causes the biased teeth 74 to be moved one toward the other so that they are no longer exposed from the window shaped openings 76 in the obturator 66, thereby presenting a smooth profile along the obturator 66. However, when the spring 72 (contained at the proximal end of the obturator handle 60) biases the latch button 64 to its extended position, the teeth 74 are exposed outwardly, and can thereby engage the distal portion 32 of the trocar cannula 28.

Therefore, as used herein the obturator 66 is inserted into the hollow cannula 28. Initially, the cannula 28 is disposed with an elongated length, so that the distal portion 32 is telescoped away from the proximal portion 30. The user then presses on the latching button 64 so that the teeth 74 are not exposed within the profile of the obturator 66. After the two handle halves 20, 60 latch one with respect to the other, the teeth 74 are placed in a position distal to the distal portion 32 of the trocar cannula 28.

Now, after the trocar 100 has been inserted into the body, the user releases the obturator handle 60 from the cannula handle 20. Thereafter, the user retracts the obturator 66 with respect to the cannula 28. Because the latch button 64 is not pressed, the teeth 74 on the latching portion 70 of the obturator 66 engage the distal portion 32 of the trocar cannula 28. Thus, during retraction, the obturator 66 itself causes retraction of the distal portion 32 with respect to the proximal portion 30 of the trocar cannula 28. After the user has derived the desired length of the trocar cannula 28, the user simply presses on the latch button 64 to cause the teeth 74 to be pulled within to the obturator 66. In this fashion, the user is then able to remove the obturator 66 without further retracting the trocar cannula 28. The trocar cannula 28 thereafter locks along the distal portion 32 with respect to the proximal portion 30, by use of the ridged notch 48 and ridges 44 contained on the exterior and interior surfaces of the distal and proximal portions 32, 30 respectively. The user has thus set the cannula 28 length to its desired size, and thus avails any desired use of cannula 28.

Of course, modifications can be made to the above described design without departing from the scope of the invention, which is to be defined by the appended claims. For instance, the relative interior and exterior positions of the proximal and distal portion of the cannula can be reversed, or the trocar obturator tip may be made sharp. Further, the sealing mechanism may be placed at different positions with respect to the proximal distal portion of the trocar cannula. These minor design modifications are inconsequential with respect to the scope of this invention.

## Claims

1. A trocar (100) comprising:
a cannula (28) for use with an obturator (66), said cannula (28) having a proximal portion (30) and a distal portion (32) extending therefrom, wherein said distal portion (32) is retractable with respect to said proximal portion (30) and
an obturator (66) insertable into said cannula (28), said obturator (66) comprising a latching mechanism (70) for retracting said distal portion (32) with respect to said proximal portion (30).

2. The trocar (100) of claim 1 further comprising a locking mechanism (48) capable of locking said proximal (30) and distal (32) portions with respect to one another.

3. The trocar (100) of claim 1 or claim 2 wherein said distal portion (32) and said proximal portion (30) have a seal (34,36) disposed between one another.

4. The trocar (100) of any preceding claim comprising an actuator mechanism (64) for actuating said latching mechanism (70).

5. The trocar (100) of claim 4 comprising a cannula handle (20) from which said cannula (28) extends and an obturator handle (60) from which said obturator (66) extends, and said latching mechanism (70) attached to said obturator.

6. The trocar (100) of claim 5 wherein said actuation mechanism (64) is attached to said obturator handle (60).

## Patentansprüche

1. Trokar (100) mit:
einer Kanüle (28) zum Gebrauch mit einem Obturator (66), wobei die Kanüle (28) einen proximalen Teil (30) und einen sich von dort erstreckenden distalen Teil (32) aufweist und der distale Teil (32) in bezug auf den proximalen Teil (30) zurückziehbar ist und
ein Obturator (66) in die Kanüle (28) einsetzbar ist, wobei der Obturator (66) einen Rastmechanismus (70) zum Zurückziehen des distalen Teiles (32) in bezug auf den proximalen Teil (30) aufweist.

2. Trokar (100) nach Anspruch 1, welcher weiterhin einen Klemmechanismus (48) aufweist, welcher derart ausgebildet ist, daß er den proximalen Teil (30) und den distalen Teil (32) in bezug zueinander fixiert.

3. Trokar (100) nach Anspruch 1 oder 2, bei welchem zwischen dem distalen Teil (32) und dem proximalen Teil (30) eine Dichtung (34, 36) angeordnet ist.

4. Trokar (100) nach einem der vorhergehenden Ansprüche mit einem Betätigungsmechanismus (64) zur Betätigung des Rastmechanismus (70).

5. Trokar (100) nach Anspruch 4 mit einem Kanülenhandgriff (20), von dem aus sich die Kanüle (28) erstreckt und einem Obturatorstiel (60), von dem aus sich der Obturator (66) erstreckt, wobei der Rastmechanismus (70) am Obturator angebracht ist.

6. Trokar (100) nach Anspruch 5, bei welchem der Betätigungsmechanismus (64) am Obturatorstiel (60) angebracht ist.

## Revendications

1. Trocart (100) comprenant:
une canule (28) à utiliser avec un obturateur (66), ladite canule (28) comportant une partie proximale (30) et une partie distale (32) se prolongeant à partir de celle-ci, ladite partie distale (32) étant rétractable par rapport à ladite partie proximale (30), et
un obturateur (66) insérable dans ladite canule (28), ledit obturateur (66) comprenant un mécanisme de verrouillage (70) pour rétracter ladite partie distale (32) par rapport à ladite partie proximale (30).

2. Trocart (100) suivant la revendication 1, comprenant en outre un mécanisme de verrouillage (48) capable de verrouiller lesdites parties proximale (30) et distale (32) l'une par rapport à l'autre.

3. Trocart (100) suivant la revendication 1 ou 2, dans lequel ladite partie distale (32) et ladite partie proximale (30) comportent un joint d'étanchéité (34, 36) placé entre elles.

4. Trocart (100) suivant l'une quelconque des revendications précédentes, comprenant un mécanisme d'actionnement (64) pour actionner ledit mécanisme de verrouillage (70).

5. Trocart (100) suivant la revendication 4, comprenant une poignée de canule (20) à partir de laquelle ladite canule (28) se prolonge, et une poignée d'obturateur (60) à partir de laquelle ledit obturateur (66) se prolonge, et ledit mécanisme de verrouillage (70) attaché audit obturateur.

6. Trocart (100) suivant la revendication 5, dans lequel ledit mécanisme d'actionnement (64) est attaché à ladite poignée de l'obturateur (60).
